# EUROPEAN PATENT SPECIFICATION

(11) **EP 4 398 826 B1**
(45) Date of publication and mention of the grant of the patent: **09.09.2026**
(21) Application number: 22793933.7
(22) Date of filing: 27.09.2022
(51) Int. Cl.: A61B 17/88, A61B 17/80, A61B 17/17, A61B 17/86

(54) **BONE ALIGNMENT SYSTEM**
KNOCHENAUSRICHTUNGSSYSTEM
SYSTÈME D'ALIGNEMENT D'OS

(30) Priority: 04.10.2021 US 202163251761 P
(43) Date of publication of application: 17.07.2024
(73) Proprietor: Smith & Nephew, Inc., Memphis. Tennessee 38116 (US); Smith & Nephew Orthopaedics AG, 6300 Zug (CH); Smith & Nephew Asia Pacific Pte. Limited, Singapore 138565 (SG)
(72) Inventor: LOUIE, Stephen, Cordova, Tennessee 38016 (US); BENNETT, Charles Ramsey, Cordova, Tennessee 38016 (US); RICCI, William, New York, New York 10075 (US)
(74) Representative: Appleyard Lees IP LLP
(86) International application number: PCT/US2022/044881
(87) International publication number: WO 2023/059482

(56) References cited:
- GB-A- 2 484 007
- US-A1- 2007 213 726
- US-A1- 2007 276 401
- US-A1- 2014 243 907
- US-A1- 2021 212 737

## Description

### CROSS-REFERENCE TO RELATED APPLICATIONS

The present application claims the benefit of the filing date of, pending U.S. provisional patent application number 63/251,761, filed October 4, 2021, entitled "Bone Alignment System and Method".

### TECHNICAL FIELD

The present disclosure is directed to a bone alignment or reduction system and method for aligning a fractured bone, and more specifically, a bone alignment or reduction system and method arranged and configured to move, realign, reposition, push, etc. a bone fragment away from a bone fixation device (e.g., bone plate). Thus arranged, in some examples, the system and method may be used to realign such as, for example, medialize, a bone fragment to produce proper reduction and alignment of the fractured bone prior to final fixation of the fractured bone, and to maintain alignment of the fractured bone during subsequent final fixation.

### BACKGROUND

Bone fractures are often repaired by securing an orthopedic implant or device to one or more patient's bone(s), bone portions, bone fragments, etc. (used interchangeably without the intent to limit or distinguish). For example, it is not uncommon for a patient to receive a bone fixation device such as, for example, a bone plate, to repair one or more fractures in a patient's bone.

During fixation or coupling of the bone alignment device (e.g., bone plate) to the patient's fractured bone, a surgeon may need to realign, reposition, or move one portion of the fractured bone relative to another portion of the fractured bone. However, coupling the bone fixation device (e.g., bone plate) to the fractured bone presents numerous challenges. For example, it is not uncommon for surgeons to fix or secure one end of the bone fixation device (e.g., bone plate) to the patient's bone such as, for example, to fix a first or proximal end of the bone fixation device (e.g., bone plate) to a first or proximal segment of the fractured bone. In addition, the surgeon may fix or secure the second or distal end of the bone fixation device (e.g., bone plate) to a second or distal segment of the fractured bone. Thereafter, the surgeon may subsequently couple the bone fixation device (e.g., bone plate) to the patient's fractured bone by inserting additional bone fixation elements (e.g., bone screws or fasteners) through one or more screw holes or openings formed in the bone fixation device (e.g., bone plate). However, during subsequent fixation of the bone fixation device (e.g., bone plate) to the patient's bone, insertion of the bone fixation elements (e.g., bone screws or fasteners) may cause the fractured bone to move closer towards the bone fixation device (e.g., bone plate). That is, tightening of the bone fixation elements (e.g., bone screws or fasteners) may cause the fractured bone to move towards the bone plate, and thus out-of-alignment.

It is with respect to these and other considerations that the present disclosure may be useful. US 2021/0212737 A1 describes implant systems such as midline incision implant systems that include an insertion guide assembly and a plate, which define fixation trajectories. GB 2484007 A describes a system for repositioning an end of a long bone comprises a bone plate and a supporting leg which can hold an arm of the bone plate inclined above a surface of the bone. US 2007/0276401 A1 describes an instrument assembly for bone drilling.

### SUMMARY OF THE INVENTION

The invention is set out in the appended claims.

Associated surgical methods are also described herein to aid understanding the invention. These methods do not form part of the claimed invention.

According to the invention, a bone alignment system arranged and configured to align a fractured bone segment comprises:
a bone plate including a plurality of screw holes;
a push rod including a proximal end and a distal end, the distal end arranged and configured to contact a patient's fractured bone segment to realign the bone segment; and
a guide arranged and configured to associate the push rod with the bone plate, the guide including a proximal end, a distal end, and a longitudinal bore extending from the proximal end to the distal end, the push rod being insertable within the longitudinal bore formed in the guide,
wherein the distal end of the guide includes external threads for threadably engaging one of the plurality of screw holes formed in the bone plate,wherein the push rod includes external threads for threadably engaging internal threads formed on a portion of the guide.

### SUMMARY OF THE DISCLOSURE

This Summary is provided to introduce a selection of concepts in a simplified form that are further described below in the Detailed Description. This Summary is not intended to identify key features or essential features of the claimed subject matter, nor is it intended as an aid in determining the scope of the claimed subject matter.

Disclosed herein is a bone alignment system arranged and configured to align a fractured bone or bone segment. In some examples, the system includes a bone alignment device such as, for example, a bone plate including a plurality of screw holes or openings, a guide, and a push rod. In use, the guide is arranged and configured to associate the push rod with the bone plate. For example, in some examples, the push rod includes a proximal end and a distal end, the distal end is arranged and configured to contact the patient's fractured bone segment to realign the bone segment. The guide includes a proximal end, a distal end, and a longitudinal bore extending from the proximal end to the distal end, the push rod being insertable within the longitudinal bore formed in the guide. In use, the guide is arranged and configured to associate the push rod with the bone plate. That is, for example, the guide may be operative coupled to the bone plate such as, for example, the guide may include an externally threaded distal end for threadably engaging one of the plurality of screw holes or openings formed in the bone plate.

In any preceding or subsequent example, the push rod may be threadably coupled to the guide. In some examples, the push rod may include external threads formed on a portion thereof, the external threads being arranged and configured to threadably engage internal threads formed on a portion of the guide. In addition, the push rod may include a blunt, sharpened, or spiked tip formed on a distal end thereof, the tip being arranged and configured to contact the bone fragment (e.g., the distal end of the push rod includes a tip arranged and configured to contact the patient's fractured bone segment).

In use, the push rod may be used to realign, reposition, move, push, etc. the bone fragment so that the bone fragment can be properly positioned. For example, in some examples, by rotating the push rod relative to the guide, the fractured bone segment can be repositioned (e.g., by threadably engaging the push rod to the guide, rotating the push rod relative to the guide causes the push rod to realign the fractured bone segment in contact with the distal end of the push rod). Thereafter, the push rod may be used to maintain the position of the fractured bone segment relative to the bone plate during subsequent fixation. That is, the push rod is arranged and configured to maintain the realigned bone fracture segment during subsequent fixation of the bone plate (e.g., once the fractured bone segment has been properly positioned, the push rod maintains the realigned position of the fractured bone segment relative to the bone plate during subsequent fixation of the bone plate to the fractured bone segment). Finally, the push rod and guide can be removed from the bone plate and an optional bone fixation element (e.g., bone fastener) can be inserted through the screw hole or opening used by the guide and the push rod to further secure the bone plate relative to the patient's bone (e.g., following removal of the push rod and the guide from the bone plate, a bone fastener can be inserted into the screw hole used by, previously occupied by, the guide and push rod).

In some examples, a method (not claimed) of aligning and securing a fractured bone segment to a bone alignment device such as, for example, a bone plate including a plurality of screw holes or openings, is disclosed. In use, the method includes realigning, repositioning, moving, pushing, etc. a bone fragment away from a bone alignment device (e.g., bone plate). Thus arranged, in some examples, the method may be used to medialize a bone fragment to produce proper reduction and alignment prior to final fixation of the fractured bone. In use, the method may be arranged and configured to enable fine (e.g., small or incremental) adjustment of the bone fragment after provisional fixation without loss of reduction. In addition, the method ensures that that proper alignment of the fractured bone is maintained during subsequent final fixation

In any preceding or subsequent example, the method may include positioning a bone plate across a patient's bone including a fracture. Next, a first end or portion of the bone plate may be secured to the patient's bone. For example, a proximal end of the bone plate may be coupled to a proximal end of the fractured bone. The bone plate may be secured using any suitable bone fixation element such as, for example, bone screws, pins, k-wires, etc. inserted through one or more screw holes or openings formed in the bone plate. Next, a second end or portion of the bone plate may be secured to the patient's bone. For example, a distal end of the bone plate may be coupled to a distal end of the fractured bone using one or more bone fixation elements.

Next, a surgeon may identify one or more bone fragments of interest requiring further reduction, alignment, etc. The one or more bone fragments may require moving the bone fragments away from the bone plate. To accomplish this, the surgeon may couple a guide to the bone plate (e.g., the surgeon may threadably engage a guide to a threaded screw hole or opening formed in the bone plate). Thereafter, the surgeon may insert a push rod into the guide until the distal end of the push rod contacts the fractured bone segment. Thereafter, the push rod may be used to move, realign, reposition, etc. the fractured bone segment (e.g., the push rod may be advanced until a sufficient separation of the fractured bone segment from the bone plate is achieved). For example, the push rod may be threadably coupled or engaged to the guide so that rotation of the push rod advances the push rod, and hence the fractured bone segment, relative to the bone plate). Thereafter, additional bone fixation elements may be inserted through the screw holes or openings formed in the bone plate to secure the bone plate to the fractured bone segment. The push rod remaining in place during subsequent fixation to maintain or hold the position (e.g., the realigned position) of the fractured bone segment relative to the bone plate and thereby prevent the fractured bone segment from being drawn in towards the bone plate during subsequent fixation of the bone plate to the fractured bone segment. Finally, the push rod and guide may be removed, and an additional bone fixation element may be secured to the fractured bone segment through the screw hole or opening used for (e.g., previously occupied by) the guide and push rod.

Exemplary embodiments of the present disclosure provide numerous advantages. For example, the system and method (the method not claimed) of the present disclosure enables medialization of a fractured bone (e.g., moving or realigning a fractured bone away from a bone plate) and subsequent maintaining the position of the fractured bone during subsequent fixation of the bone plate to the fractured bone. That is, the system and method of the present disclosure enable a surgeon to move or realign a fractured bone away from a bone plate. In addition, the system and method enables a surgeon to maintain the realigned position of the fractured bone while the bone plate is being secured to the fractured bone, and thus prevents the fractured bone from being moved or drawn towards the bone plate during subsequent fixation of the bone plate to the fractured bone. As such, the system and method of the present disclosure enables simplified mobilization of a bone fragment, while enabling the surgeon to maintain reduction of the fractured bone.

Further features and advantages of at least some of the exemplary embodiments of the present invention, as well as the structure and operation (the operation not claimed) of various exemplary embodiments of the present invention, are described in detail below with reference to the accompanying drawings.

### BRIEF DESCRIPTION OF THE DRAWINGS

By way of example, specific examples of the disclosed device will now be described, with reference to the accompanying drawings, in which:
**FIG. 1** is a perspective view of an example of a bone alignment system in accordance with one or more features of the present disclosure, the bone alignment system including a bone fixation device (e.g., a bone plate), a guide, and a push rod;
**FIG. 2** is an exploded, perspective view of the bone alignment system shown in **FIG. 1****;**
**FIG. 3** is a cross-sectional view of an example of the guide shown in **FIG. 1****;**
**FIG. 4** is a side view of an example of the push rod shown in **FIG. 1****;** and
**FIGS. 5-8** illustrate various views of a method using the bone alignment system of **FIG. 1****.**

It should be understood that the drawings are not necessarily to scale and that the disclosed exemplary embodiments are sometimes illustrated diagrammatically and in partial views. In certain instances, details which are not necessary for an understanding of the disclosed methods and devices or which render other details difficult to perceive may have been omitted. It should be further understood that this disclosure is not limited to the particular exemplary embodiments illustrated herein. In the drawings, like numbers refer to like elements throughout unless otherwise noted.

### DETAILED DESCRIPTION

Various features or the like of a bone alignment or reduction (terms used interchangeably herein without the intent to limit or distinguish) system, and corresponding method, arranged and configured to move, align, reposition, push, etc. (terms used interchangeably herein without the intent to limit) a fractured bone and to maintain the position of the fractured bone during final fixation will now be described more fully hereinafter with reference to the accompanying drawings, in which one or more features of the bone alignment system and method will be shown and described.

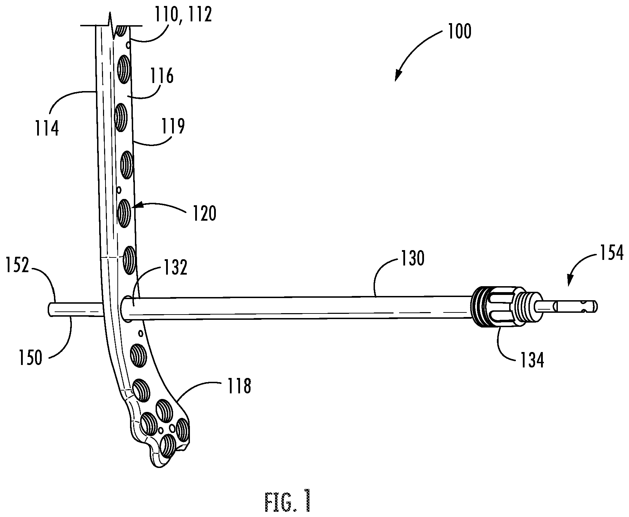

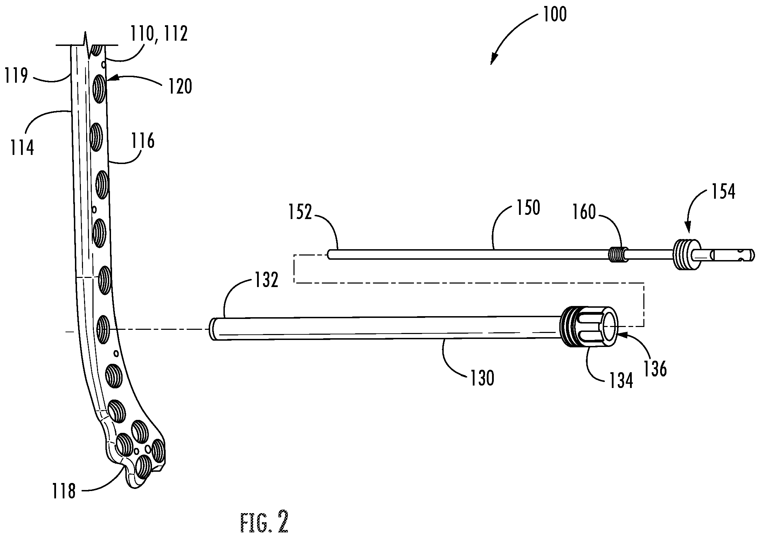

Referring to **FIGS. 1** and **2****,** an example of a bone alignment system 100 in accordance with one or more features of the present disclosure is illustrated. As illustrated, the bone alignment system 100 includes a bone fixation device 110, a guide 130, and a rod, a push rod, a push screw, or the like 150 (terms used interchangeably herein without the intent to limit or distinguish). Alternatively, in some non-claimed examples, the guide is optional and the push rod 150 may be threadably coupled directly to the bone fixation device 110.

In use, the bone fixation device 110 may be any suitable device now known or hereafter developed for fixing or coupling to a fractured bone. As illustrated, in some examples, the bone fixation device 110 may be a bone plate 112. In use, the bone plate 112 is arranged and configured for positioning adjacent to a patient's bone such as, for example, a fractured bone. As will be described and generally shown herein, the bone plate 112 may be coupled to a patient's fractured femur, although the present disclosure is not so limited, and the bone plate may be provided in any suitable shape and/or configuration, which, as will be appreciated by one of ordinary skill in the art, may be dependent on the location and type of patient's bone being fixed. For example, the bone plate may include a bone conforming arcuate surface. In addition, the bone plate may be arranged and configured to span, contact, etc. a distal femur, a proximal femur, a distal tibia, a proximal tibia, a proximal humerus, a distal humerus, a fibula, an ulna, a radius, a distal radius, bones of the foot, or bones of the hand, shaft fractures on long bones, etc.

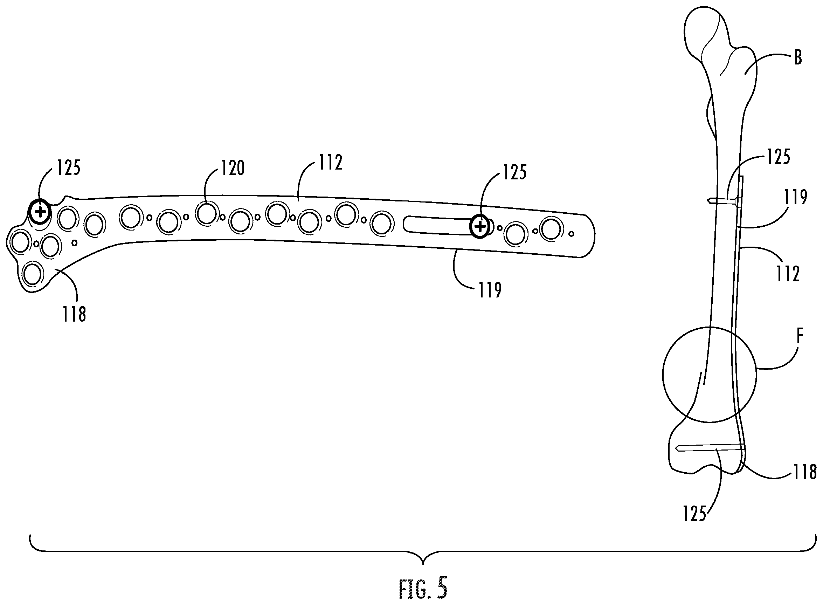

As illustrated, the bone plate 112 includes a lower or bone facing surface 114 and an upper surface 116. In addition, the bone plate 112 includes a head portion 118 and a shaft portion 119. Moreover, the bone plate 112 includes a plurality of screw holes or openings 120 (terms used interchangeably herein without the intent to limit or distinguish) formed therein for receiving a plurality of fasteners or screws 125 (FIGS. 5-8) for coupling the bone plate 112 to the patient's bone.

In some examples, the screw holes 120 are in the form of a locking screw (or fastener) opening. That is, as will be appreciated by one of ordinary skill in the art, locking screw openings include a plurality of threads formed on an inner surface thereof for mating with threads formed on an outer surface of a head portion of a bone fastener. Thus arranged, the bone fastener may be said to be locked to the bone plate 112 via the locking screw openings. That is, as will be appreciated by one of ordinary skill in the art, the bone fastener is threaded through one of the locking screw openings formed in the bone plate 112 and into the patient's bone. The bone fastener is secured to the bone plate 112 via threads formed on the head portion of the bone fastener that cooperate with the threaded locking screw opening formed in the bone plate 112. This secures the bone plate 112 with respect to the patient's bone and provides rigid fixation between the bone plate 112 and the bone fasteners. That is, because the head portion of the bone fastener interdigitates with the threads formed in the locking screw openings of the bone plate 112, the bone plate 112 and the fasteners form a stable system or construct, and the stability of the fracture can be dependent on or aided by the stiffness of the construct. Locking a bone fastener into the bone plate 112 can achieve angular and axial stability and eliminate the possibility for the bone fastener to toggle, slide, or be dislodged, reducing the risk of postoperative loss of reduction. However, in non-claimed examples, the screw holes 120 may have other configurations such as, for example, variable angled openings, which are non-threaded and enable the bone fastener to be angled relative to the bone plate.

As will be appreciated by one of ordinary skill in the art, the number of screw holes 120 can be variable depending on the length of the plate. In addition, and/or alternatively, the bone plate 112 may be manufactured from any suitable material now known or hereafter developed, including, for example, metals, polymers, plastics, ceramics, resorbable, non-resorbable, composite materials, etc. Suitable materials may include, for example, titanium, stainless steel, cobalt chrome, polyetheretherketone (PEEK), polyethylene, ultra-high molecular weight polyethylene (UHMWPE), resorbable polylactic acid (PLA), polyglycolic acid (PGA), combinations or alloys of such materials or any other appropriate material that has sufficient strength to be secured to and hold bone, while also having sufficient biocompatibility to be implanted into a patient's body. In some examples, the bone fastener may be manufactured from the same material as the bone plate. In other examples, the fasteners may be manufactured from a different material as compared to the bone plate.

The fastener can be any type of fastener now known or hereafter developed. For example, the fastener may include any type of external thread including standard or non-standard threads. For example, the external threads can be arranged as a continuous ridge or a non-continuous ridge. The external threads can form a portion of a revolution, one complete revolution, multiple revolutions, a single lead, multiple leads, or any other threads known in the art. Additionally, and/or alternatively, in the case of locking screws, the head portion of the fastener can include any surface that will engage with and seat within a locking screw opening formed in the bone plates. For example, the head portion can include threads. Alternatively, the head portion can include a series of dimples, ridges, bumps, textured areas, or any other surface that can secure the fastener.

The fastener may be any fastener now known or hereafter developed, made out of any appropriate material now known or hereafter developed. The fastener may include a bore for receiving a driver in order to drive the fastener through the bone fixation plate and into the patient's bone. The bore may be any size and shape, for example, it may have a hexagonal configuration to receive a corresponding hexagonal driver, a Phillips screw head, a flat-head, a star configuration, Torx, or any other appropriate configuration that can cooperate with a driver to drive the fastener through the bone plate and into the patient's bone.

The shaft of the fastener may be fully threaded, partially threaded, or a helical blade, and/or may include one or more tacks, deployable talons, expandable elements, or any feature that allows the shaft to engage the patient's bone. It is also possible that shaft be non-threaded so that the fastener takes the form of a peg or a pin. The end of the shaft may be a self-tapping or self-drilling tip.

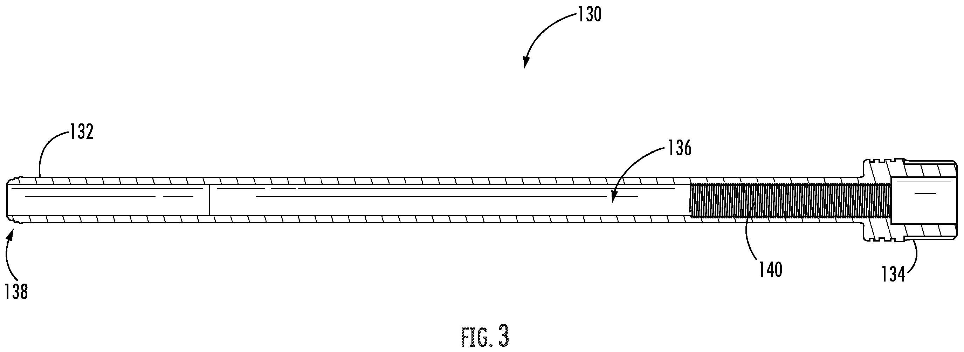

Referring to **FIGS. 1-3****,** the guide 130 includes a first end 132, a second end 134 and a cannulated bore 136 extending between the first and second ends 132, 134. As best illustrated in **FIG. 3****,** the first end 132 of the guide 130 includes a coupling mechanism formed on the end thereof for engaging the bone plate 112. As illustrated, the coupling mechanism is in the form of external threads 138 formed at the first end 132 of the guide 130 for engaging the internally threaded screw holes 120 formed in the bone plate 112, although this is but one configuration and the guide 130 may include alternate coupling mechanisms (not claimed) for engaging the bone plate. In addition, as illustrated, the cannulated bore 136 of the guide 130 may include a partially, internally threaded region 140. As will be described below, in use, the internal threads 140 of the guide 130 are arranged and configured to threadably engage the push rod 150.

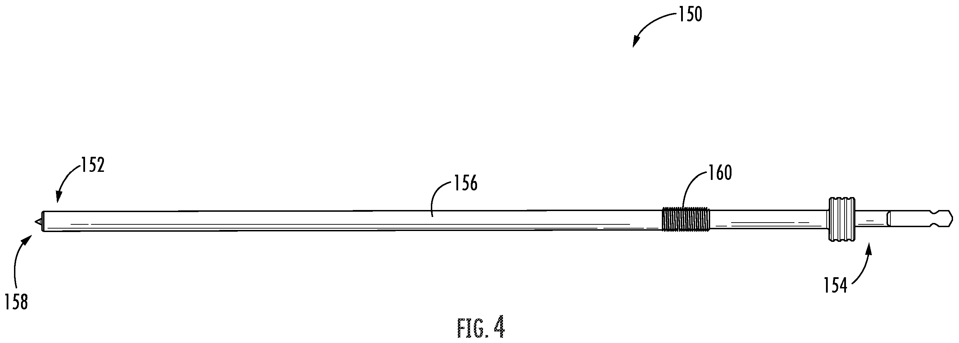

Referring to **FIGS. 1****,** **2****,** and **4****,** the push rod 150 includes a first end 152, a second end 154 and a shaft 156 extending between the first and second ends 152, 154. As best illustrated in **FIG. 4****,** in some examples, the first end 152 of the push rod 150 includes a blunt, sharpened, or spiked tip 158 for contacting, engaging, etc. the patient's bone. By providing a sharpened or spike tip 158, the push rod 150 is better able to capture the patient's bone fragment. In addition, as illustrated, the shaft 156 of the push rod 150 includes a partially, externally threaded region 160. As will be described below, in use, with the guide 130 coupled to the bone plate 112, the push rod 150 may be inserted into the cannulated bore 136 of the guide 130. The push rod 150 may be advanced until the first end (e.g., distal end) 152 of the push rod 150 contacts the patient's bone. For example, the push rod 150 may be slidably inserted into the cannulated bore 136 of the guide 130 until the external threads 160 formed on the push rod 150 contact the internal threads 140 formed in the guide 130. Thereafter, during use, interaction of the external threads 160 of the push rod 150 and the internal threads 140 of the guide 130 enables the surgeon to perform fine or smaller adjustments of the push rod 150 relative to the guide 130, and thus fine or smaller adjustments of the patient's bone relative to the bone plate 112. Thus arranged, the surgeon can realign the patient's bone relative to the bone plate 112 and maintain the position of the realigned bone relative to the bone plate 112 during subsequent final fixation of the bone plate 112 to the patient's bone. In particular, by advancing the push rod 150, the surgeon can move the patient's bone away from the bone plate 112 and maintain the position of the realigned bone relative to the bone plate 112 during subsequent final fixation of the bone plate 112 to the patient's bone. This is particularly useful when a fractured bone of the patient needs to be medialized, as will be described in greater detail below.

With reference to **FIGS. 5-8****,** an example of a method for realigning a patient's fractured bone in accordance with one or more features of the present disclosure will be described. As generally shown, in some examples, the bone alignment system 100 may be used to realign a patient's distal femur B. With reference to **FIG. 5****,** the proximal end of the bone plate 112 may be coupled to the patient's bone B (e.g., femur). In addition, the distal end of the bone plate 112 may be coupled to the distal end of the patient's bone B (e.g., femur). The bone plate 112 being coupled to the patient's bone B using one or more bone fixation elements shown as bone fasteners or screws 125. As illustrated, the patient's bone B is fractured F between the proximal and distal ends such that the bone plate 112 spans the fracture F. In addition, as illustrated, the patient' bone B may require medialization (e.g., to properly align the fractured bone, one portion or segment of the patient's fractured bone may need to be moved or pushed away from the bone plate 112) to prevent and/or correct a "hockey stick" deformity.

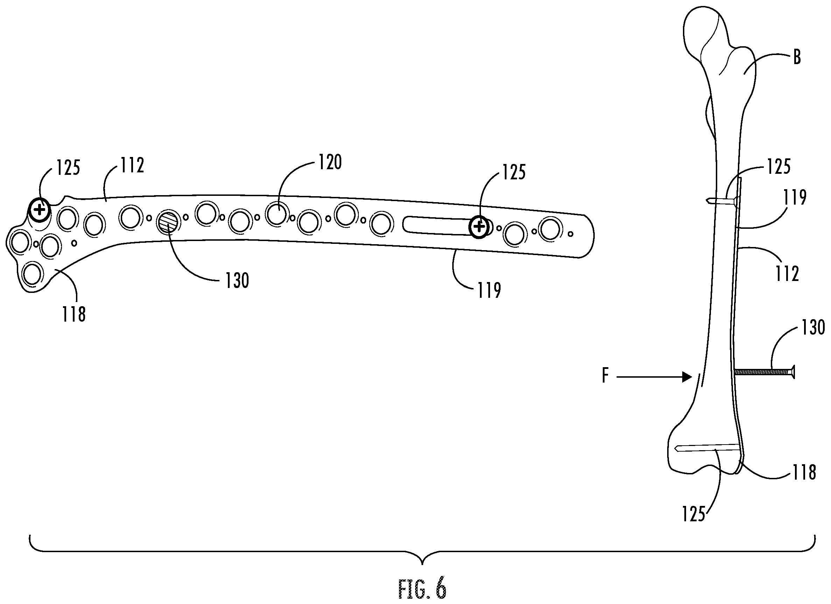

With reference to **FIG. 6****,** with provisional fixation of the proximal and distal ends of the bone plate 112 to the fractured bone B, the guide 130 may be coupled (e.g., threadably coupled) to the bone plate 112 using one of the plurality of screw holes 120 formed in the bone plate 112. As illustrated, in some examples, the guide 130 is positioned in one of the screw holes 120 positioned adjacent to, such as, for example, proximally, of the bone fracture F since the distal end of the fractured bone B is often permanently fixed to the bone plate 112 using a plurality of bone fasteners or screws 125 positioned through the head portion 118 of the bone plate 112. Moreover, as illustrated, the guide 130 is preferably positioned in one of the screw holes 120 positioned nearest the end of the bone fracture F. Thus arranged, the push rod 150 can be used to move or push an end such as, for example, the distal end of the proximal fragment of the patient's bone.

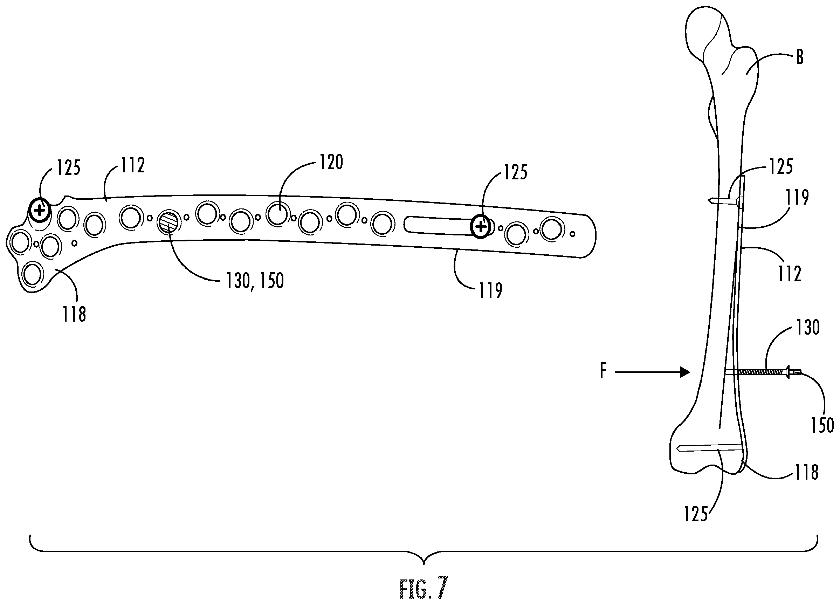

With reference to **FIG. 7****,** with the guide 130 coupled to the bone plate 112, the push rod 150 is inserted into the cannulated bore 136 of the guide 130 and advanced until the first end (e.g., distal end) 152 of the push rod 150 contacts and pushes the distal end of the proximal bone fragment away from the bone plate 112 and reduces the fracture F as desired by the surgeon.

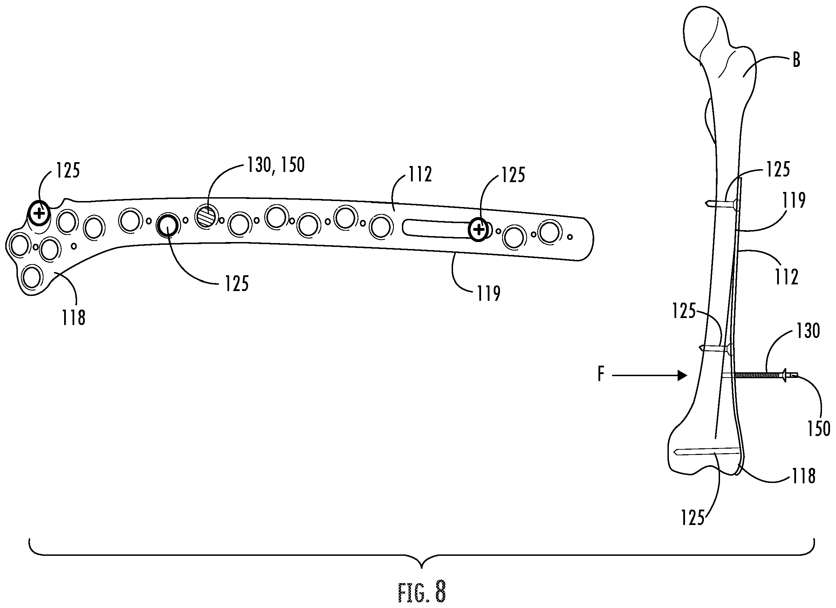

With reference to **FIG. 8****,** with the push rod 150 contacting the distal end of the proximal fragment of the patient's bone B, additional bone fasteners or screws 125 can be inserted through the bone plate 112 and into engagement with the patient's bone B to fixedly couple the bone plate 112 to the bone B. By maintaining the position of the push rod 150 during subsequently fixation of the bone plate 112 to the patient's bone B, the distal portion of the proximal bone fragment is prevented from moving closer to the bone plate 112 during screw fixation. As such, the realigned position of the fractured bone is maintained during final fixation of the bone plate 112.

Finally, the push rod 150 and the guide 130 can be removed. Optionally, a final bone fastener or screw 125 can be coupled to the patient's bone B using the screw hole used to couple the guide 130 to the bone plate 112.

In accordance with the features of the present disclosure, as described herein, in use, the push rod enables a surgeon to maintain reduction and/or realignment of the fractured bone fragment during final fixation of the bone plate to the fractured bone. For example, the system and method enable a surgeon to medialize a bone fragment in order to produce proper reduction and alignment prior to final fixation of the fractured bone to a bone plate. As such, the system and method helps eliminate so-called "hockey stick" deformities. In addition, the system and method enables further adjustment of the fractured bone relative to the bone plate after provisional fixation of the bone plate to the fractured bone and without loss of reduction by allowing the surgeon to advance the push rod or retract the push rod, respectively. In addition, the system and method ensures alignment of the fractured bone is maintained during subsequent final fixation.

While specific exemplary embodiments of the present disclosure have been described, alternate configurations are envisioned. In a non-claimed example, it is envisioned that the system and method may utilize a pneumatic or hydraulic piston or a 4-bar mechanism to facilitate linear actuation of the fractured bone away from the bone plate. In a non-claimed alternative, it is envisioned, that the system and method may utilize a cannulated threaded rod arranged and configured to be threaded over a k-wire.

The foregoing description has broad application. Accordingly, the discussion of any example or embodiment is meant only to be explanatory and is not intended to suggest that the scope of the claims is limited to these examples or embodiments.

The term "a" or "an" entity, as used herein, refers to one or more of that entity. As such, the terms "a" (or "an"), "one or more" and "at least one" can be used interchangeably herein. The use of "including," "comprising," or "having" and variations thereof herein is meant to encompass the items listed thereafter as well as additional items. Accordingly, the terms "including," "comprising," or "having" and variations thereof are open-ended expressions and can be used interchangeably herein. The phrases "at least one", "one or more", and "and/or", as used herein, are open-ended expressions that are both conjunctive and disjunctive in operation.

All directional references (e.g., proximal, distal, upper, underside, lower, upward, downward, left, right, lateral, longitudinal, front, back, top, bottom, above, below, vertical, horizontal, radial, axial, clockwise, and counterclockwise) are only used for identification purposes to aid the reader's understanding of the present disclosure, and do not create limitations, particularly as to the position, orientation, or use of this disclosure. Connection references (e.g., attached, coupled, connected, and joined) are to be construed broadly and may include intermediate members between a collection of elements and relative movement between elements unless otherwise indicated. As such, connection references do not necessarily infer that two elements are directly connected and in fixed relation to each other. Identification references (e.g., primary, secondary, first, second, third, fourth, etc.) are not intended to connote importance or priority but are used to distinguish one feature from another. The drawings are for purposes of illustration only and the dimensions, positions, order, and relative sizes reflected in the drawings attached hereto may vary.

## Claims

1. A bone alignment system (100) arranged and configured to align a fractured bone segment, the bone alignment system comprising:
a bone plate (112) including a plurality of screw holes (120);
a push rod (150) including a proximal end (154) and a distal end (152), the distal end arranged and configured to contact a patient's fractured bone segment to realign the bone segment; and
a guide (130) arranged and configured to associate the push rod with the bone plate, the guide including a proximal end (134), a distal end (132), and a longitudinal bore (136) extending from the proximal end to the distal end, the push rod being insertable within the longitudinal bore formed in the guide,
**characterized in that**
the distal end of the guide includes external threads (138) for threadably engaging one of the plurality of screw holes formed in the bone plate,
wherein the push rod includes external threads (160) for threadably engaging internal threads formed on a portion of the guide.

2. The bone alignment system of claim 1, configured such that rotation of the push rod relative to the guide to which the push rod is threadably engaged is operable to cause the push rod to realign the fractured bone segment.

3. The bone alignment system of claim 2, wherein, once properly positioned, the push rod is operable to maintain a realigned position of the fractured bone segment relative to the bone plate during subsequent fixation of the bone plate to the fractured bone segment.

4. The bone alignment system of claim 2, wherein following removal of the push rod and the guide from the bone plate, the screw hole of the bone plate used by the guide is configured to receive a bone fastener (125), the bone fastener being configured to secure the bone plate relative to the patient's bone.

5. The bone alignment system of claim 1, wherein the distal end of the push rod includes a tip (158) arranged and configured to contact the patient's fractured bone segment.

## Patentansprüche

1. Knochenausrichtungssystem (100), das zum Ausrichten eines frakturierten Knochensegments angeordnet und ausgelegt ist, wobei das Knochenausrichtungssystem Folgendes umfasst:
eine Knochenplatte (112) mit mehreren Schraubenlöchern (120);
eine Schubstange (150) mit einem proximalen Ende (154) und einem distalen Ende (152), wobei das distale Ende angeordnet und ausgelegt ist, um ein frakturiertes Knochensegment eines Patienten zu berühren, um das Knochensegment neu auszurichten; und
eine Führung (130), die angeordnet und ausgelegt ist, um die Schubstange mit der Knochenplatte in Verbindung zu bringen, wobei die Führung ein proximales Ende (134), ein distales Ende (132) und eine sich vom proximalen Ende zum distalen Ende erstreckende Längsbohrung (136) aufweist, wobei die Schubstange in die in der Führung gebildete Längsbohrung einführbar ist,
**dadurch gekennzeichnet, dass**
das distale Ende der Führung Außengewinde (138) aufweist, um eines der mehreren in der Knochenplatte gebildeten Schraubenlöcher in Gewindeeingriff zu nehmen,
wobei die Schubstange Außengewinde (160) aufweist, um an einem Abschnitt der Führung gebildeten Innengewinde in Eingriff zu nehmen.

2. Knochenausrichtungssystem nach Anspruch 1, das so ausgelegt ist, dass eine Drehung der Schubstange bezüglich der Führung, mit der die Schubstange in Gewindeeingriff steht, in der Lage ist zu bewirken, dass die Schubstange das frakturierte Knochensegment neu ausrichtet.

3. Knochenausrichtungssystem nach Anspruch 2, wobei die Schubstange nach richtiger Positionierung bedienbar ist, um eine neu ausgerichtete Position des frakturierten Knochensegments bezüglich der Knochenplatte während einer anschließenden Fixierung der Knochenplatte an dem frakturierten Knochensegment aufrechtzuerhalten.

4. Knochenausrichtungssystem nach Anspruch 2, wobei nach Entfernen der Schubstange und der Führung von der Knochenplatte das von der Führung verwendete Schraubenloch der Knochenplatte zur Aufnahme eines Knochenbefestigungselements (125) ausgelegt ist, wobei das Knochenbefestigungselement ausgelegt ist, um die Knochenplatte bezüglich des Knochens des Patienten zu befestigen.

5. Knochenausrichtungssystem nach Anspruch 1, wobei das distale Ende der Schubstange eine Spitze (158) aufweist, die angeordnet und ausgelegt ist, um das frakturierte Knochensegment des Patienten zu berühren.

## Revendications

1. Système d'alignement d'os (100) agencé et configuré pour aligner un segment d'os fracturé, le système d'alignement d'os comprenant :
une plaque osseuse (112) comprenant une pluralité de trous de vis (120) ;
une tige de poussée (150) comprenant une extrémité proximale (154) et une extrémité distale (152), l'extrémité distale étant agencée et configurée pour entrer en contact avec un segment d'os fracturé d'un patient afin de réaligner le segment d'os ; et
un guide (130) agencé et configuré pour associer la tige de poussée à la plaque osseuse, le guide comprenant une extrémité proximale (134), une extrémité distale (132) et un alésage longitudinal (136) s'étendant de l'extrémité proximale à l'extrémité distale, la tige de poussée pouvant être insérée dans l'alésage longitudinal formé dans le guide,
**caractérisé en ce que**
l'extrémité distale du guide comprend des filets externes (138) destinés à venir en prise par vissage avec l'un de la pluralité de trous de vis formés dans la plaque osseuse,
la tige de poussée comprenant des filets externes (160) destinés à venir en prise par vissage avec des filets internes formés sur une partie du guide.

2. Système d'alignement d'os selon la revendication 1, configuré de telle sorte que la rotation de la tige de poussée par rapport au guide sur lequel la tige de poussée est engagée par vissage peut être actionnée pour amener la tige de poussée à réaligner le segment d'os fracturé.

3. Système d'alignement d'os selon la revendication 2, une fois correctement positionnée, la tige de poussée pouvant être actionnée pour maintenir une position réalignée du segment d'os fracturé par rapport à la plaque osseuse pendant la fixation ultérieure de la plaque osseuse au segment d'os fracturé.

4. Système d'alignement d'os selon la revendication 2, après le retrait de la tige de poussée et du guide de la plaque osseuse, le trou de vis de la plaque osseuse utilisé par le guide étant configuré pour recevoir une attache osseuse (125), l'attache osseuse étant configurée pour fixer la plaque osseuse par rapport à l'os du patient.

5. Système d'alignement d'os selon la revendication 1, l'extrémité distale de la tige de poussée comprenant une pointe (158) agencée et configurée pour venir en contact avec le segment d'os fracturé du patient.
